Europäisches Patentamt

European Patent Office

Office européen des brevets

(11) Publication number: **0 306 466**
**A2**

# (12) EUROPEAN PATENT APPLICATION

(21) Application number: **88850289.5**

(22) Date of filing: **01.09.88**

(51) Int. Cl.⁴: **C 12 M 1/36**

(30) Priority: **04.09.87 DK 4635/87**

(43) Date of publication of application:
**08.03.89 Bulletin 89/10**

(84) Designated Contracting States:
**AT BE CH DE ES FR GB GR IT LI LU NL SE**

(71) Applicant: **DANSK BIOPROTEIN A/S**
**Kaerbygade 14 Vester Kaerby**
**DK-5320 Agedrup (DK)**

(72) Inventor: **Jorgensen, Lars**
**Gerdaslundsvej 3**
**DK-5230 Odense M (DK)**

(74) Representative: **Wiklund, Ingrid Helena et al**
**AWAPATENT AB P.O. Box 5117**
**S-200 71 Malmö (SE)**

(54) **Method and means for the production of a micro-organism cell mass.**

(57) By ensuring that the concentration of dissolved methane (11) and oxygen (13) and ammonium is constant throughout the whole of the fermentor (1), a considerable increase is achieved in both the production capacity of the biomass as well as its uniformity and purity. To this is added a lower production cost, in that the cell density in the fluid can be doubled, and also a reduction in the consumption of gas.

Furthermore, the formation of nitrite can be controlled with the help of a nitrite meter, thus ensuring against the formation of nitrite becoming too great and thus hampering the growth.

In order to achieve the greatest possible mixing of methane and air before its introduction into the fermentor, this takes place in one or more ejectors (33) which are driven by means of pressure fluid. Mechanical mixing arrangements can hereby be avoided.

Finally, the supply is effected via nozzles (36), the injection direction of which can be adjusted for the greatest possible turbulance and therewith the mixing in the fermentor.

Fig.1

EP 0 306 466 A2

**Description**

## METHOD AND MEANS FOR THE PRODUCTION OF A MICRO-ORGANISM CELL MASS

The invention relates to a method for the production of a micro-organism cell mass in a fermentation tank, where the bacteria cultivated is methanotropic, where the carbon and energy source is natural gas, where the oxygen source is air, where the nitrogen source is ammonia, and where the cultivation can take place under septic conditions, said fermentation process being regulated by means of a computer, and also to means for the execution of the method.

It is known, among others from the description of English patent no. 1.270.006, to produce a biomass in a fermentation tank by the cultivating of micro-organisms in a fluid growth medium containing assimilative nitrogen sources and essential mineral salts in the presence of methane gas, said organisms being extracted from the fluid growth medium.

These hitherto known methods have not, however, functioned satisfactorily, in that it is particularly difficult under septic conditions to avoid the growth of undesired toxic and/or growth-imparing organisms, and it is also difficult to achieve a completely pure and uniform biomass.

Therefore, success has not hitherto been attained in achieving a continuous biomass production which is profitable.

The object of the invention is to make this biomass production profitable, and this is achieved by a method by which the concentration of dissolved methane and oxygen in the fermentor is held constant throughout the whole of the fermentor.

There is hereby achieved a hitherto unknown degree of stability and therewith uniform optimum growth conditions for the bacteria culture. This optimization of the process provides the possibility of a high cell density in the fluid, namely right up to more than 4.5%. This is twice the amount when compared with the hitherto known level, which means a saving in the consequent drying process, where only a half of the water thus needs to be removed.

This enables a uniform high production, which in practice has shown to be up to ten times greater than that which the hitherto known methods have been capable of producing.

In addition to this, the amount of gas which is added is only that necessary to maintain the culture's optimum activity. This results in a considerably lower gas consumption when compared with the hitherto known methods, which entail a considerable loss of gas for reasons of the great amount of discharge gas which is derived from the fermentor.

This contributes towards making the process profitable, the reason being that the price of the gas constitutes around 75% of the cost of production.

By using mass spectrometry, as presented in claim 2, the control can be effected on-line.

As presented in claim 3, by using mass spectrometry with membrane inlet, the measurements can be effected in a quick and reliable manner.

As presented in claim 4, by holding the concentration of ammonia constant throughout the whole of the fermentor, a rapid growth of the bacteria can be ensured.

As presented in claim 5, by using an ion-selective electrode, the measurement can be effected continuously.

As presented in claim 6, by the additional use of a nitrate meter, it can be controlled that the formation of nitrate in the fermentor does not become too great and thus impair the growth.

As presented in claim 7, by mixing methane and air in the ejectors, one can achieve particularly high gas transfer constants for reasons of the considerable absorption of oxygen which is achieved in the ejector.

As presented in claim 8, by using fluid under pressure as pump medium, a high degree of dispersion is ensured without the use of mechanical stirring arrangements.

As presented in claim 9, by using adjustable nozzles, these can afford any desired direction of flow within the fermentor.

Finally, as presented in claim 10, it is expedient for the direction of the nozzle to be adjusted by being able to be turned.

In the following, the invention will be described in closer detail with reference to the drawing, where

fig. 1 shows in schematic form a system for the execution of the method,

fig. 2 shows a section of the lower part of the fermentor with a pipe system and an ejector seen in direction II-II in fig. 4.

fig. 3 shows a sectional drawing of the fermentor seen in the direction III-III in fig. 2 and 4, and

fig. 4 shows a sectional drawing of the fermentor seen in the direction IV-IV in fig. 2 and 3.

fig. 5 shows, in graph form, how the speed of dilution varies with the methane concentration.

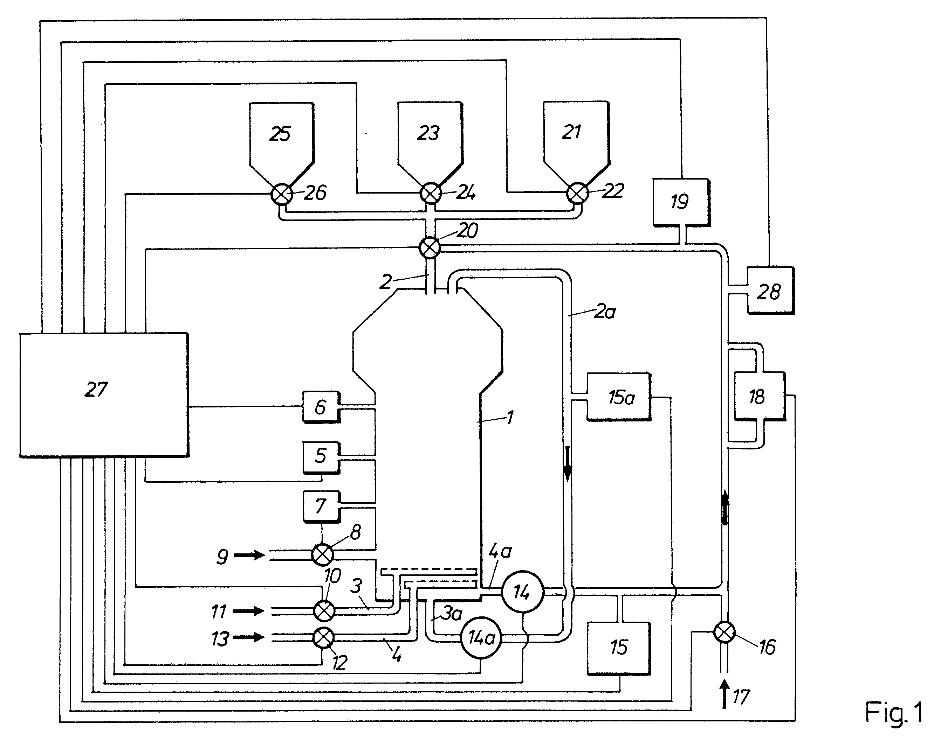

In fig. 1 is shown an embodiment of a system in which the fermentor is a so-called "air-lift" fermentor comprising a fermentation tank 1.

The fermentor can be configured with a closed top as a pressure tank, whereby the solubility of the nutrient gases can be increased. The solubility of the gases is proportional to their partial pressure.

The supply of natural gas 11 and oxygen 13 takes place via adjustable valves 10 and 12 respectively to the bottom of the tank through distribution pipes 3 and 4, while a nutrient solution 23 is fed to the top of the tank via regulation valves 24 and 20.

Natural gas and oxygen are introduced separately via two distribution pipes 3 and 4 in the bottom of the fermentor. Each distribution pipe consists of a pipe arrangement which is evenly distributed over the bottom area. In the top of the pipes there are 0.5mm to 1mm holes through which the gas flows out.

The distributor system generates gas bubbles with a diameter of less than 1cm at 0.5mm hole diameters, evenly distributed over the fermentor's cross-sectional area.

The transfer of the supplied natural gas and oxygen to the fermentation medium takes place in the tank when the gas bubbles are blown in from the tank's bottom. The tank can be divided into a first section in which the fluid moves upwards as a result of a high air content, and a second section in which the fluid moves downwards as a result of a lower air content. In order to ensure a homogeneous mixing of the culture and an optimum utilization of the blow-in gases, the fermentor must be of considerable height, the reason being that the gases must pass up to fifty metres of fluid before they are completely utilized.

In order to reduce this long fluid passage and hereby reduce the costs, the fermentor can be provided, as shown in the drawing, with a circulation which connects the tank's lower pipe 3a with its top via 2a by means of an outer bypass with circulation pump 14a. In this way it is possible to force circulate the gases through the medium in the tank until the desired absorption has taken place.

The number of recirculations and injections of new gases 11 and 13 is regulated depending on the content of gases remaining in the recirculated air.

For the determination of the methane and oxygen content in the circulation, a mass spectrometer 15a is introduced.

The dwell-time of the gases in the fermentor is increased by circulating the fluid opposite to the gas flow. A flow of fluid is taken from the bottom of the tank via the pipe 14a, and by means of a circulation pump 14 this is fed up to the top of the fermentor, where it is introduced together with the supply of nutrient fluid via pipe 2. The fluid circulation also contributes towards providing a good mixing in the fermentor. Together with the supply of nutrient fluid, the circulated fluid can be fed to the top of the fermentor via a spray or grid, whereby a foam suppression effect is achieved.

Alternatively, the circulating fluid can be introduced together with the recirculating gas under pressure via a downwards-directed nozzle placed in the fermentation medium. A particularly good transfer of the gases to the fluid can be achieved.

A mass spectrometer 6 is connected to the tank 1 for the determination of methane and oxygen content in the medium inside the fermentor 1. By using a mass spectrometer 6 in the fermentor and a mass spectrometer 15 for measurements in the circulated fluid, and with knowledge of the transport time in the circulation, the metabolic activity can be calculated on the basis of the difference in the registered values in and outside the fermentor.

A mass spectrometer with membrane inlet is used for the continuous measurement of dissolved methane and oxygen in the fermentor. The supply of gas is regulated on the basis of mass spectrometric measurements, so that the concentration of dissolved methane and oxygen in the medium is constant.

For the circulation, a spectrophotometer 18 is also inserted for the determination of the cell density in the medium. This is effected by the admission of a known light source which is sent through the medium and dispersed by the bacteria, after which the amount of light through the bacteria is determined as a measure of the cell density. This method is influenced by air bubbles which, like the bacteria, have a light diffusion effect. However, the problem can be solved by pumping a part of the fermentation medium through a spectrophotometer with a cylindrical glass tube which sits vertically as cuvette. The pump flow through the vertical cylinder is stopped at suitable intervals of time. After a few seconds, the air bubbles will have risen to the upper end of the cylinder and a measurement of the micro-organisms existing in the lower end of the cylinder can be carried out without any influence by the original con tent of gas. When the measurement has been made, the pump flow is started again, whereby new fermentation medium is brought to the measuring cell. The pump and the registration of the measuring result are controlled by a computer 27, and are thus automated.

In the event of the fermentation being desired to be carried out at constant cell density, new medium 17 can be pumped into the circulation and in the fermentor concurrently with the growth of the cells, whereby the cell density can be held constant. By registering the rate at which new medium 17 is added to the fermentation, the speed of fermentation and thus the specific growth rate can be determined.

In a corresponding manner as for the cell density, the metabolic activity can control the speed of dilution via the valve 16, so that the activity of the culture can be held completely constant.

The source of nitrogen in the form of ammonia 25 is introduced into the medium via a valve 26. Since the ammonia has a restrictive influence on the methane oxidation, a controlled supply of ammonia is necessary in order to achieve optimum growth. Ammonia ($NH_3$) in an aqueous medium will be in equilibrium with the ammonium ion ($NH_4+$). At pH 6.8, where methanotrophic bacteria thrive well, most of the ammonia will be in ammonium form. Ammonium ions can be measured with an ion-selective electrode 19.

The electrode can be placed in the fermentor 1 itself or, as shown, externally in connection with the circulating flow from the fermentor. Ammonium-selective electrodes are unable to withstand autoclavation, and therefore do not find immediate application in aseptic fermentations. On the basis of the measurement of the concentration of ammonium, a computer 27 can control the addition of ammonia 25. This addition is an expression for the consumption of ammonium, and consequently also a measure of the metabolic activity of the culture.

An ion-selective electrode 19 is used for the continuous measurement of ammonium in the fermenter.

3

Furthermore, a nitrite sensor 28, build after the well-known FIA (Flow Injection Analysis) principle, is used for the measurement of nitrite in the fermentor. The addition of medium and ammonium is regulated on the basis of ammonium measurements, so that the concentration of ammonium in the fermentor is held constant. It is very important that the ammonium concentration does not vary. Ammonium is a prerequisite for the rapid growth of the bacteria, but at the same time it hampers the growth. The latter is due to ammonium being a competitive substrate for the methane-oxidizing enzyme, which can oxidize ammonium to nitrite. The formation of nitrite depends on the concentration of ammonium and methane in the medium.

A process without control of the supply of ammonium shows instability. A quite small increase in the ammonium concentration hampers the methane oxidation, and therewith the cells. This leads to a reduced consumption of ammonium, and if the supply of ammonium is not immediately reduced or stopped, the result is an increase in the concentration of ammonium, the formation of nitrite and further restriction of the growth etc.

If not controlled, the build-in instability in a steady-state culture leads to self-destruction. Only by a continuous measurement and control of the ammonium concentration can this self-destruction be prevented.

The measurement of the ammonium concentration is used to hold a constant biomass in the fermentor, and to regulate the rate of dilution so that the productivity becomes optimal. The contents of nitrogen in the biomass, bound in the form of proteins, is quite constant around 11% or less, and the relationship between the content of nitrogen in the feed flow in the form of ammonium and the ammonium concentration under steady-state in the fermentor, determines the biomass concentration in the fermentor.

A modified FIA (Flow Injection Analysis) system is used for nitrate measurements. The same is removed from the fermentor across a dialysis membrane which is selective for smaller molecules and does not allow cells to pass. The membrane is kept clean on the fermentor side by a strong flow. On the analysis side, the nitrite is absorbed in a dialysis flow which is fed slowly across the membrane. The flow is then mixed with a colour reagent which reacts specifically with nitrite, and the absorption is measured as an expression for the amount of nitrite in the medium. For calibration of the signal, a change is made between measuring of the dialysis flow and a known standard.

The formation of nitrite is an indication that the process is not being effected in an optimum manner. This can be because the concentration of ammonium has become too high, that the methane concentration is too low, or that there is not sufficiently good stirring in the fermentor.

The measurements can be combined with the methane and oxygen absorption rate measurements and the cell measurements for an integrated control of the fermentation process.

The pH of the culture can be controlled via a pH electrode 5 placed in the fermentor 1 and the addition of acid or base 21 via a valve 22.

The temperature in the fermentor is determined with a temperature sensor 7 in the fermentor, and the supply of cooling water 9 to the fermentor 1 is regulated via a valve 8.

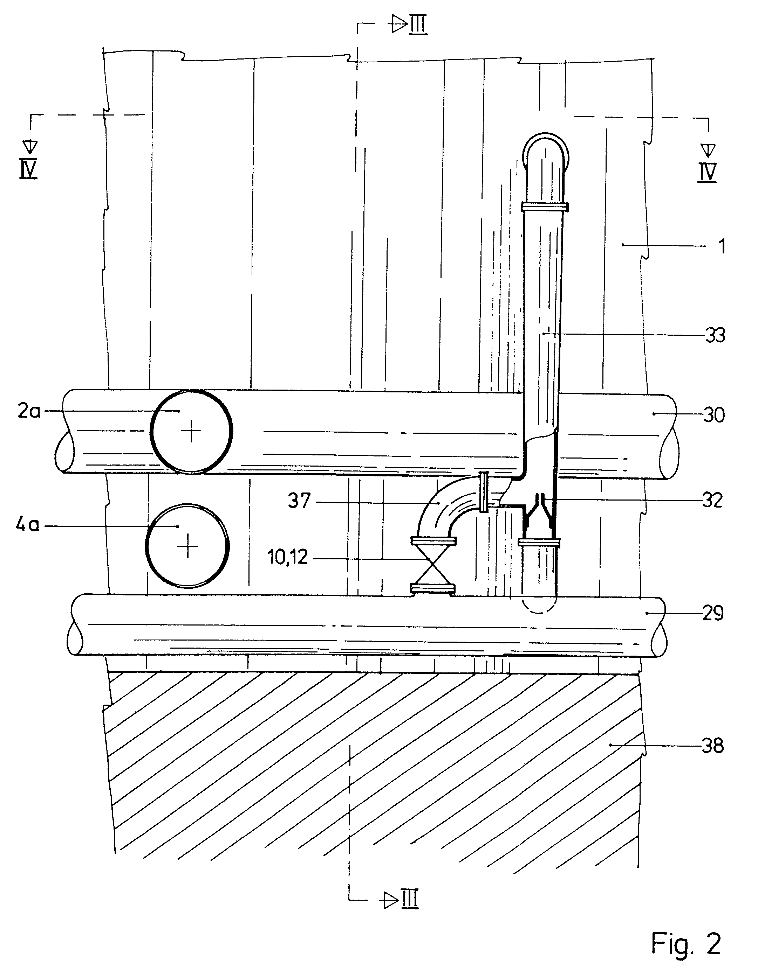

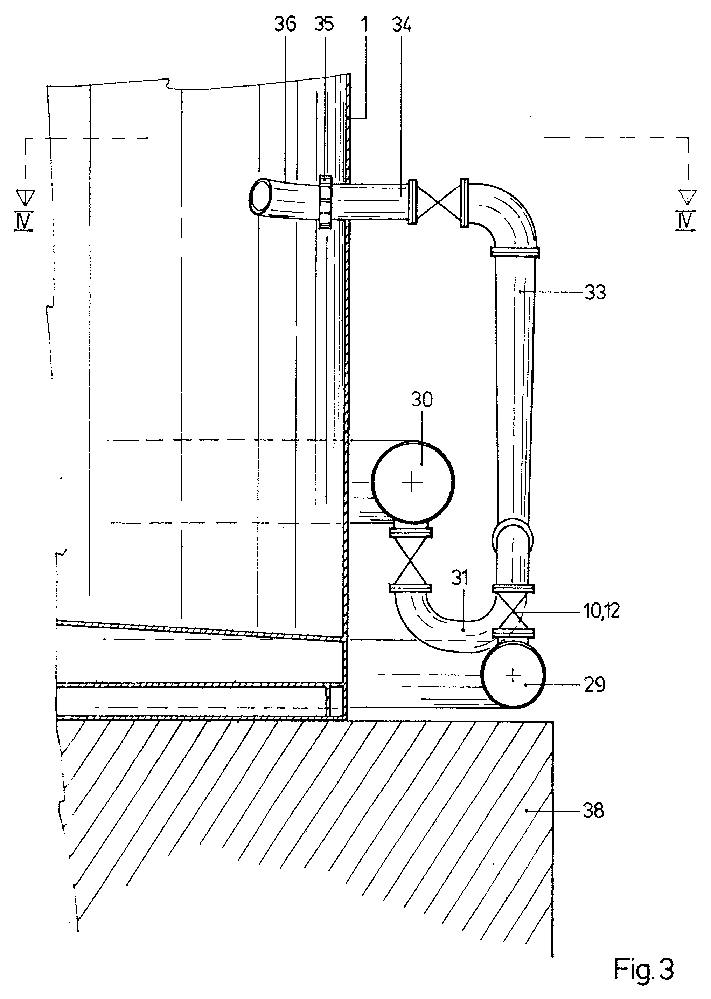

In fig. 2-4 is shown another embodiment of means for the addition of methane and oxygen. These means comprise two pipe systems which extend around the fermentor 1, and which comprise pipes 3, 4 and 29 for methane 11 and air 13, and hereto connected distributor pipes 37, each of which leads into its ejector 33.

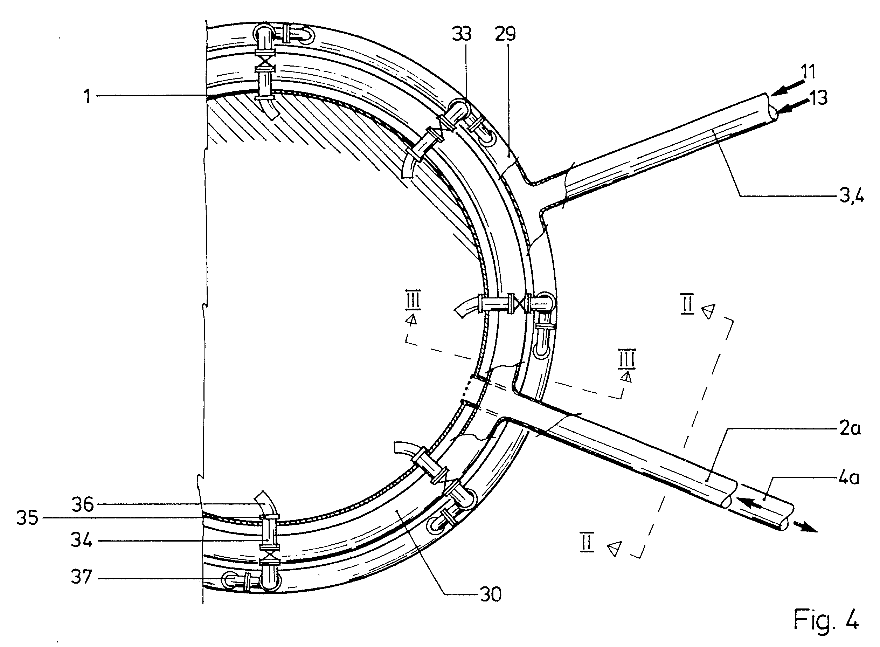

In addition, there is a fluid supply pipe 2a and 30 which leads fluid under pressure at about 4 atm to distributor pipe 31 which leads into the ejector nozzle 32. The gases are hereby sucked into the medium under great turbulence. The discharge into the fermentor takes place via nozzle mouths 36, each of which is attached in a pivotable manner to an adjustable assembly element 35, which is firmly connected to the distributor pipe 34. Each individual nozzle 36 can hereby be set, as shown in fig. 4, in that direction which provides the best degree of mixing in the tank.

In the following, three examples of the use of the method according to the invention will be described.

Example 1

The methane-oxidating culture is grown in a 2-litre fermentor under stirring (1500 rpm). The supply of natural gas and oxygen is regulated on the basis of mass spectrometric measurements of dissolved methane and oxygen. The ammonium concentration is measured by an ammonium-selective electrode, and is held constant at about 1 mM. Medium and ammonium are added continuously in the same ratio, so that the cell density is held constant. By using different steady-state concentrations of dissolved oxygen and methane, varying growth rate and loss of methane/oxygen in the discharge gas is achieved.

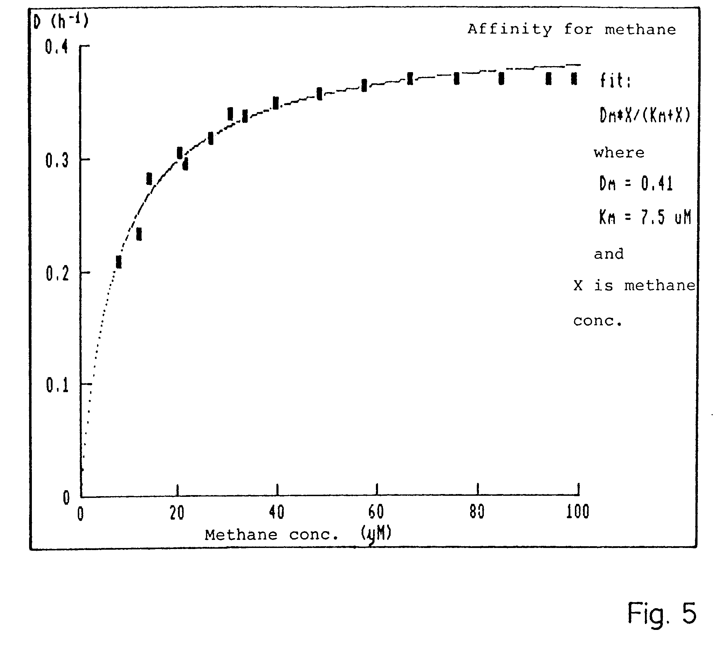

Fig. 5 shows, in an experiment where 1 g/l $NH_3NO_3$ and a steady-state cell density of approx. 3 g drystuff/l are used, how the dilution rate varies the medium's methane concentration. The optimum dilution rate of 0.37 per hour is achieved at methane concentrations of 60 uM and above. Correspondingly, only some few uM oxygen are required in the medium in order to achieve maximum dilution rate. Table 1 shows the dilution rate and the loss of methane and oxygen to the discharge air at different dilution rates. It is seen that in order to reduce the loss as much as possible, it is important to hold as low as possible the concentrations of dissolved methane and oxygen.

Table 1

| Concentration | | dilution | loss in discharge gas | |
|---|---|---|---|---|
| methane | oxygen | rate | methane | oxygen |
| uM | uM | $h^{-1}$ | % | % |
| 100 | 20 | 0.37 | 25 | 28 |
| 60 | 20 | 0.37 | 19 | 20 |
| 60 | 2 | 0.33 | 17 | 5 |
| 20 | 4 | 0.3 | 11 | 7.3 |

Example 2

An injector nozzle is used for the mixing of natural gas and air in a 700-litre fermentor. A mass spectrometer is used fcr the control of the gas supply, and an ammonium-selective electrode and nitrite sensor for the control of medium supply. This system has given a productivity which is 3-4 times higher than that achieved in a corresponding air-lift fermentor. With a cell density of 25 g drystuff/l and a dilution rate of 0.2 per hour, the culture demands a supply of a little less than 6 $m^3$ natural gas and 42 $m^3$ air per hour. Because of the poor solubility of methane and oxygen, this large gas requirement can be accommodated if either a special injector nozzle as described here is used, or a system in which the fermentor operates under several atmospheric pressure. The latter gives rise to safety problems.

Table 2

The productivity in 700 l fermentor operated as a traditional air-lift fermentor and with injector nozzle.

| Cell density g/l | Dilution rate $(t^{-1})$ | Productivity g/l/hour |
|---|---|---|
| 4 | 0.33 | 1.3 |
| 8 | 0.12 | 0.96 |
| 16 | 0.02 | 0.32 |

With nozzle:

| 4 | 0.35 | 1.4 |
|---|---|---|
| 8 | 0.33 | 2.6 |
| 16 | 0.27 | 4.32 |
| 26 | 0.21 | 4.9 |
| 30 | 0.18 | 5.4 |

Example 3

With 8 nozzles placed in an outer ring around the fermentor, a 50,000 litre fermentor is used for the mixing of methane gas and air, cf. fig. 2-4. The injection into the fermentor can be regulated by an external adjustment of the mouths of the nozzles, which can be turned. The nozzles can thus be adjusted to give the optimum gas transfer constant and stirring in the 50,000 l capacity tank. For reasons of the effective mixing of gases in the tank and the control used for the gas and medium supply, high production of around 6g biomass/l/hour can be achieved.

Claims

1. Method for the production of a micro-organism cell mass in a fermentation tank, where the cultivated bacteria are methanotrophic, where the carbon and energy source is natural gas, where the oxygen source is air, where the nitrogen source is ammonia, and where the cultivation can take place under septic conditions, said fermentation process being regulated by means of a computer, **characterized** in that the concentration of dissolved methane (11) and oxygen (13) is held constant throughout the whole of the fermentor (1).

2. Method according to claim 1, **characterized** in that the concentration is measured by mass spectrometry.

3. Measuring equipment for the execution of the method according to claim 2, **characterized** in that it consists of a mass spectrometer (6, 15, 15a) with membrane inlet.

4. Method according to claims 1 and 2, **characterized** in that the ammonium concentration is held constant throughout the whole of the fermentor (1).

5. Measuring equipment for the execution of the method according to claim 4, **characterized** in that it includes an ion-selective measuring electrode (19).

6. Measuring equipment according to claim 5, **characterized** in that it further includes a nitrite meter (28) with dialysis membrane.

7. Method according to claim 1, 2 and 4, **characterized** in that methane (11) and air (13) are mixed in one or more ejectors (33) before the mixture is fed to the fermentor (1) (fig. 2-4).

8. Ejector for the execution of the method according to claim 7, **characterized** in that fluid under pressure is fed to the ejector nozzle (32) as drive medium.

9. Ejector according to claim 8, **characterized** in that the discharge is effected through a nozzle (34, 35, 36) having an outflow direction in the fermentor (1) which can be adjusted.

10. Ejector according to claim 9, **characterized** in that the mouth part (36) of the nozzle extends in relation to the inlet pipe (34) on which it is pivotally mounted (35).

Fig. 1

Fig. 2

Fig. 3

Fig. 4

EP 0 306 466 A2

Fig. 5